# FASCICULE DE BREVET EUROPEEN

(11) **EP 0 854 126 B1**
(45) Date de publication et mention de la délivrance du brevet: **11.10.2000**
(21) Numéro de dépôt: 98400046.3
(22) Date de dépôt: 13.01.1998
(51) Int. Cl.: C07C 15/24, C07C 7/00

(54) **Procédé de purification de naphtalène par hydrotraitement sélectif suivi d'une séparation**
Verfahren zur Reinigung von Naphthalin durch selektive Hydrierung und nachfolgende Trennung
Process for the purification of naphthalene by selective hydrotreatment followed by a separation

(30) Priorité: 20.01.1997 FR 9700618
(43) Date de publication de la demande: 22.07.1998
(73) Titulaire: INSTITUT FRANCAIS DU PETROLE, 92500 Rueil Malmaison (FR); BEFS PROKEM, 68071 Mulhouse CEDEX (FR)
(72) Inventeur: Mignard, Samuel, 78400 Chatou (FR); Drouglazet, Guenael, 682600 Kingersheim (FR); Kasztelan, Slavik, 92500 Rueil Malmaison (FR); Cosyns, Jean, 78580 Maule (FR); Bloch, Michel, 92500 Rueil Malmaison (FR); Genin, René, 68200 Mulhouse (FR)
(74) Mandataire: Andreeff, François

(56) Documents cités:
- CHEMICAL ABSTRACTS, vol. 119, no. 9, 30 août 1993 Columbus, Ohio, US; abstract no. 95910m, page 1054; XP000404533 -& JP 05 085 960 A (OSAKA GAS CO) 6 avril 1993

## Description

L'invention concerne un procédé de purification d'une coupe naphtalène, généralement issue de la carbochimie.

Parmi les produits issus de la distillation des goudrons de houille se trouve une fraction naphtalénique contenant essentiellement du naphtalène mais on note également, de façon non limitative, la présence de produits soufrés tel que par exemple le benzothiophène, des produits azotés telle que par exemple la quinoleine, des produits oxygénés tel que par exemple des dérivés phénoliques mais aussi des hydrocarbures insaturés tels que l'indène.

Actuellement, il existe deux types de naphtalène sur le marché. Le premier type est un "naphtalène technique" dont la pureté est supérieure à 98%. Le deuxième type est un "naphtalène pur" principalement utilisé pour la synthèse et la fabrication de produits anti-insectes (la "naphtaline"). Comme ce produit doit être parfaitement incolore, il doit être pur. La pureté doit être supérieure à 99,95% et la teneur en soufre pratiquement nulle (quelques ppm poids).

La coupe naphtalène utilisable dans le procédé selon l'invention peut contenir plus de 50% poids de naphtalène, avantageusement plus de 75%, et le plus souvent plus de 85% poids. Elle contient également des composés soufrés représentant jusqu'à 5% poids de soufre mais le plus souvent moins de 1%, des composés azotés sous forme par exemple de quinoleine (jusqu'à 1 % poids), des monooléfines comme, par exemple de l'indène (jusqu'à 1% poids mais le plus souvent moins de 0.5%) et des composés oxygénés comme par exemple les phénols (jusqu'à 1% poids mais le plus souvent moins de 0.5% poids).

La présente invention concerne un procédé de purification d'une coupe naphtalène, en particulier d'une coupe de naphtalène issue de la carbochimie pour obtenir un un "naphtalène technique".

Des procédés de traitement de coupes naphtalène existent déjà.

Ainsi, la demande de brevet JP-05-017,376 décrit un procédé d'hydrogénation du naphtalène à basse pression (0-20 bars) dans lequel les impuretés sont hydrogénées ainsi qu'une partie du naphtalène et une partie de la tétraline formée.

Une autre demande JP-05-085,960 décrit un procédé de purification d'une coupe naphtalène comprenant une première étape d'hydrogénation en phase liquide sous faible ou moyenne pression (0-20 bars), à 100-300°C, avec un catalyseur choisi dans le groupe formé par les catalyseurs Ni-Co-Mo, platine sur charbon, Pt-Ni-Mo, Pd-alumine, CoMo-alumine. L'effluent obtenu est, dans une seconde étape, dégazé pour éliminer H₂S, NH₃ et l'éthylbenzène. Puis, l'effluent est lavé avec un acide inorganique (troisième étape) et ensuite séparé (quatrième étape). L'effluent ainsi obtenu est déshydraté par distillation azéotropique avec l'éthylbenzène (cinquième étape). Les impuretés résiduelles sont adsorbées (sixième étape) par de l'argile. L'effluent peut alors être distillé (septième étape) et pressé (huitième étape) pour obtenir le naphtalène purifié.

Les demanderesses ont recherché un procédé de traitement de coupes naphtalène qui soit simple à mettre en oeuvre, au contraire du brevet JP-05-085,960 nécessitant de nombreuses étapes de traitement et qui permette d'obtenir des rendements plus élevés en naphtalène que dans le brevet JP-05-017,376.

L'invention proposée est basée sur l'utilisation d'un catalyseur particulier permettant un hydrotraitement préalable de la charge poussé, de façon à éliminer en une seule étape la plus grande partie des impuretés soufrées, azotées, oxygénées, oléfiniques tout en limitant l'hydrogénation (réduction de la quantité de tétraline et de décaline) ce qui constitue un hydrotraitement sélectif de façon à pouvoir, uniquement par cristallisation, séparer le naphtalène purifié. Plus précisément, l'invention a pour objet un procédé de traitement de coupe naphtalène contenant des impuretés soufrées et/ou azotées et/ou oxygénées et/ou oléfiniques, dans lequel, dans une première étape, la coupe naphtalène est mise au contact, en présence d'hydrogène, d'un catalyseur contenant au moins une matrice, au moins un élément du groupe VIII et au moins un élément du groupe VI. Il est caractérisé en ce que le catalyseur de première étape utilisé contient 5-40% poids d'oxydes de métaux des groupes VIII et VI, avec un rapport pondéral en oxyde de métal du groupe VI sur oxyde de métal du groupe VIII compris entre 1.25 et 20, ledit catalyseur présentant une surface spécifique BET d'au plus 220 m²/g, un volume poreux compris entre 0.35 et 0.7 ml/g et un diamètre moyen de pores d'au moins 10 nm, et que la coupe naphtalène est mise au contact dudit catalyseur à une température de 150-325°C, sous une pression totale de 0.1 MPa-0.9 MPa, avec une vitesse volumique horaire de 0.05-10 h⁻¹ et le rapport molaire hydrogène/naphtalène est compris entre 0.1 et 1.3, de façon à ce que le rendement en tétraline soit inférieur à 10% poids et dans une seconde étape, une partie au moins de l'effluent issu de la première étape sont séparés de H₂S, NH₃ et l'eau et dans une troisième étape, une partie au moins de l'effluent issu de la seconde étape est soumise à un traitement permettant de séparer dudit effluent le naphtalène et la tétraline. Si l'on utilise le recyclage de la tétraline, le rapport pondéral tétraline/naphtalène est compris entre 0.005 et 0.08.

Le procédé comprend trois étapes successives. La première étape a pour but d'abaisser la teneur en soufre présent sous forme de molécules soufrées à la valeur désirée. Les molécules soufrées sont alors transformées en molécules désulfurées et H₂S. Dans cette première étape, les molécules azotées sont largement transformées en molécules déazotées et NH₃. Elle permet aussi d'hydrogéner les molécules oléfiniques et de dehydroxyler les molécules contenant un groupement OH. Au cours de cette étape, le catalyseur et les conditions opératoires sont choisis de façon à réaliser un hydrotraitement poussé tout en limitant l'hydrogénation du naphtalène en tétraline. En effet, la tétraline peut être ici considérée comme un sous produit de la réaction qui est alors indésirable. Il ne se forme pratiquement pas de tétraline (généralement moins de 5% poids dans les effluents et de préférence moins de 3%).

Par hydrotraitement poussé, on comprend une désulfuration d'au moins 70% et de préférence d'au moins 90% et de façon encore plus préférée d'au moins 98%, une déazotation d'au moins 50% et de façon préférée d'au moins 80%, une hydrogénation des oléfines d'au moins 80% et de préférence d'au moins 95% et une déhydroxylation d'au moins 75% et de préférence d'au moins 90%.

La pression totale est comprise entre 0.1 MPa (1 bar) et 0.9 MPa (9 bars) et de façon préférée entre 0.2 MPa (2 bars) et 0.9 MPa (9 bars) et de façon encore plus préférée entre 0.2 MPa (2 bars) et 0.8 MPa (8 bars). La température de réaction est comprise entre 150°C et 325°C et de façon préférée entre 200°C et 320°C et de façon encore plus préférée entre 220°C et 300°C. La vitesse volumique horaire (VVH) est comprise entre 0.05 h⁻¹ et 10 h⁻¹ et de façon préférée entre 0.1 h⁻¹ et 5 h⁻¹ et de façon encore plus préférée entre 0.15 h⁻¹ et 2 h⁻¹. Le rapport molaire hydrogène/naphtalène à l'entrée du réacteur est compris entre 0.1 et 1.3 et de façon préférée inférieure à 1.

Le catalyseur utilisé pour la première étape d'hydrotraitement est un catalyseur contenant au moins une matrice avantageusement à base d'alumine, de préférence ne contenant pas de zéolithe, et au moins un métal ayant une fonction hydro-déshydrogénante. Ladite matrice peut également renfermer de la silice-alumine, de l'oxyde de bore, de la magnésie, de la zircone, de l'argile ou une combinaison de ces oxydes. La fonction hydro-déshydrogénante est assurée par une combinaison d'au moins un métal ou composé de métal du groupe VIII tels que le nickel et le cobalt notamment et d'au-moins un métal ou composé de métal du groupe VI (notamment le molybdène ou le tungstène) de la classification périodique des éléments. La concentration totale en oxydes de métaux des groupes VI et VIII est comprise entre 5 et 40 % en poids et de préférence entre 7 et 30 % en poids et le rapport pondéral (exprimé en oxyde métallique de métal) (ou métaux) du groupe VI sur métal (ou métaux) du groupe VIII est compris entre 1.25 et 20 et de préférence entre 2 et 10. De plus, ce catalyseur peut de préférence contenir du phosphore. La teneur en phosphore, exprimée en concentration en oxyde de phosphore P₂O₅, sera inférieure à 15 % en poids et de préférence inférieure à 10 % en poids.

De façon préférée, le catalyseur utilisé est de type CoMo déposé sur alumine. Le catalyseur présente des caractéristiques très particulières :
- la surface spécifique BET est d'au plus 220 m²/g, voire inférieure à 200 m²/g lorsque le catalyseur contient du phosphore, et dans ce cas là, la surface préférée est d'au plus 180 m²/g.
- le volume poreux est compris entre 0.35 et 0.7 ml/g
- le diamètre moyen des pores est d'au moins 100Å (10nm) et de préférence compris entre 100 et 200 Å (10 et 20nm), ce qui signifie que la fraction du volume poreux correspondant aux pores de diamètre inférieur à 90 Å (9 nm) est faible (0-15%).

Avant de passer sur la troisième étape, une partie au moins de l'effluent ayant subi l'hydrotraitement sera pratiquement débarrassé en totalité ou en partie (d'au moins 95% et mieux d'au moins 99%) des molécules de H₂S, NH₃ et H₂O générées. Toute méthode permettant de réaliser ces séparations est utilisable. De façon usuelle, une simple colonne de strippage sera utilisée.

La troisième étape a pour but de récupérer la plus grande quantité de naphtalène avec la pureté la plus élevée possible. Toute méthode de séparation peut être utilisée, par exemple la distillation. La pureté du naphtalène est alors de l'ordre de 99.6%. Pour obtenir une pureté supérieure (par exemple, supérieure à 99.96%), il est particulièrement avantageux d'utiliser un procédé de purification en milieu fondu, et en particulier le procédé PROAB® commercialisé par la société BEFS PROKEM. On connaît notamment par le brevet FR-2.493.172 son procédé et l'appareil pour la purification de tout produit cristallisable. L'appareil, qui a ensuite été amélioré (demande de brevet EP-A-728.508), peut être utilisé de façon très avantageuse pour ce type de purification. Cette troisième étape aura essentiellement pour rôle de séparer le naphtalène pur de la tétraline et autres impuretés contenues dans l'effluent provenant de l'étape précédente.

Le procédé de cristallisation permet la séparation du mélange obtenu après hydrogénation et strippage. Le procédé, ayant une haute efficacité sur les composants constituant les impuretés du naphtalène pur, permet d'atteindre la haute pureté. Il est utilisé seul, contrairement aux autres procédés actuellement en service où une combinaison de 2, 3 voire plus différents procédés est nécessaire pour atteindre la haute pureté indiquée au tableau 3, par exemple distillation puis extraction à l'acide, traitement à l'argile, évaporation.

Il peut cependant être avantageux de faire précéder l'étape de cristallisation d'une étape de distillation, le procédé opérant alors en fait en quatre étapes.

Le procédé de cristallisation peut utiliser les techniques de lavage des cristaux de naphtalène pur soit par la technique de fusion fractionnée soit par la technique du lavage à l'aide d'une partie du produit purifié qui est alimenté à contre-courant du procédé de purification.

L'effluent strippé est soumis à un traitement par cristallisation se déroulant de la façon suivante :
les opérations de cristallisation s'effectuent dans un appareil entièrement automatisé sans aucune intervention manuelle. Un fluide circule dans l'appareil. Selon les phases du procédé, ce fluide est alternativement chauffé ou refroidi par l'intermédiaire d'échangeurs de chaleur. Une régulation fine permet d'apporter avec exactitude au naphtalène les calories ou frigories nécessaires à l'opération. Le naphtalène brut, préalablement fondu pour être introduit dans l'un des cristalliseurs, est soumis à une loi de descente en température sous inertage, ce qui provoque une cristallisation très contrôlée du produit. Au fur et à mesure du refroidissement, la quantité de cristaux augmente jusqu'à un certain taux prédéterminé ; solide et liquide sont alors séparés par égouttage. Pour parfaire la purification des cristaux, on procède :
- soit à une fusion partielle des cristaux (ressuage). En fondant, les cristaux génèrent un naphtalène pur qui se mélange au film résiduel de liquide et le purifie,
- soit à du lavage consistant en des séquences de remplissage/vidange de l'appareil par des liquides de plus en plus purs.

On passe ensuite à la phase de la fusion totale des cristaux purs résiduels puis à la vidange de l'appareil. Suivant l'efficacité du procédé de cristallisation choisi et de la pureté finale désirée, un ou plusieurs étages de cristallisation peuvent être utilisés. D'une façon générale, le procédé de cristallisation en milieu fondu opère à des températures comprises entre 60-90°C et de préférence 75-85°C par cristallisation du naphtalène dans des enceintes remplies ou partiellement remplies. Il peut opérer de façon dynamique, par exemple avec circulation du produit à cristalliser à l'intérieur des tubes ou par ruissellement en film à l'intérieur ou à l'extérieur de tubes. Il peut également fonctionner en mode statique, par exemple au moyen d'une capacité remplie du produit à cristalliser munie d'éléments de transfert de chaleur plongeant dans le produit.

Performances du procédé de cristallisation : le procédé de cristallisation en milieu fondu permet d'atteindre de hautes puretés en naphtalène (99-99,999 %) et plus généralement supérieures à 99,90 %.

De façon usuelle, le rendement en tétraline est inférieur à 10% poids et de façon préférée inférieure à 5% poids ou mieux inférieure ou égale à 3% poids (le rendement étant la masse de tétraline formée par rapport à la masse de naphtalène à la sortie du réacteur d'hydrotraitement).

De façon à augmenter le rendement en naphtalène, il est également possible de réaliser le recyclage de la tétraline c'est-à-dire d'ajouter tout ou partie de la tétraline récupérée après l'étape de séparation à la charge de départ. La quantité de tétraline introduite est dans le rapport pondéral tétraline/naphtalène compris entre 0.005 et 0.08.

Les travaux de recherche effectués par la demanderesse l'ont conduit à découvrir que, d'une façon surprenante, l'utilisation de l'enchaînement hydrotraitement sélectif et poussé puis séparation permettait d'obtenir un naphtalène de haute pureté avec un rendement très élevé.

Les exemples suivant illustrent l'invention sans toutefois en limiter la portée. Ils sont conduits sur une charge de naphtalène de type carbochimique dont l'analyse est donnée dans le tableau 1.

**Tableau 1**

| | Teneur (%poids) |
|---|---|
| Benzothiophène soit soufre | 2,45 soit 0.586 |
| Naphtalène | 96,5 |
| Tétraline | 0 |
| Phénols | 0,3 |
| Indène | 0,15 |
| 1 et 2 méthyl-naphtalène | 0,2 |
| Quinoleine | 0,3 |

### Exemple 1 : Hydrotraitement (HDT) puis strippage et cristallisation

La purification de la charge a été réalisée par un hydrotraitement sélectif poussé suivi d'un strippage puis d'une cristallisation.

Les conditions opératoires de l'hydrotraitement sont les suivantes : température : 318°C, pression totale : 0.5 MPa (5 bars), débit d'hydrogène : 125 l/l soit un rapport molaire de 0.62 et VVH : 1 h⁻¹.

Le catalyseur utilisé contient 3%poids de CoO et 14% poids de MoO₃ déposé sur alumine. La surface spécifique du catalyseur est de 210 m²/g, le diamètre moyen des pores est égal à 110 Å et le volume poreux égal à 0.58 ml/g.

La totalité de l'effluent est soumis à un strippage de façon à éliminer en phase gazeuse H₂S, NH₃ et H₂O. L'indène hydrogéné totalement reste dans l'effluent.

Dans le tableau 2, nous avons reporté l'analyse de l'effluent ainsi obtenu après hydrotraitement et strippage. L'effluent obtenu après strippage est ensuite soumis au traitement de cristallisation précédemment décrit réalisé dans un cristalliseur statique de type PROABD® utilisant la fusion partielle des cristaux pour purifier le naphtalène. Le naphtalène préalablement fondu est introduit dans le cristalliseur. Ce dernier est ensuite soumis à une descente en température provoquant une cristallisation contrôlée du produit. On interrompt la cristallisation lorsque la température du produit atteint 66°C, afin d'égoutter les eaux mères. Après égouttage, on procède à une fusion partielle des cristaux, en remontant la température jusqu'à 78,5°C. Le produit est alors complètement fondu. Pour atteindre une pureté finale de 99,96 %, un deuxième étage de purification par cristallisation est nécessaire. Il comporte les mêmes opérations que celles décrites ci-dessus, mais les températures opératoires sont différentes :
- cristallisation jusqu'à 78°C puis égouttage des eaux mères
- fusion partielle des cristaux jusqu'à 79,8°C
- fusion totale du produit purifié.

L'analyse de l'effluent ainsi obtenu après hydrotraitement puis strippage puis cristallisation est également reportée dans le tableau 2.

**Tableau 2**

| | HDT+strippage | HDT + strippage + cristallisation |
|---|---|---|
| Soufre (ppm poids) | 23 | 10 |
| Naphtalène (%pds) | 97.0 | 99.960 |
| Tétraline (%pds) | 2.7 | 0.025 |
| Décaline (%poids) | 0.05 | < 0.001 |
| Phénols (%poids) | < 0.001 | < 0.001 |
| Indène (%poids) | < 0.001 | < 0.001 |
| 1&2 méthyl-naphtalène (%poids) | 0.20 | 0,005 |
| Quinoleine (%poids) | 0.10 | 0,004 |
| Point de fusion (°C) | 78 | > 80.2 |

L'utilisation de l'hydrotraitement sélectif et de la cristallisation permet d'obtenir un produit de très bonne qualité. La teneur en soufre est abaissée à 10 ppm poids ce qui correspond à une hydrodésulfuration supérieure à 99.8%. Avec une pureté de 99.96% et un point de fusion de plus de 80.2°C, le naphtalène obtenu est de type "naphtalène pur".

### Exemple 2 : utilisation du recyclage

Dans l'exemple précédent, le produit final ne contient pratiquement pas de tétraline car celle-ci a été éliminée au cours de l'étape de cristallisation. La tétraline séparée est recyclée pratiquement en totalité à l'entrée du réacteur ce qui permet d'améliorer le rendement en naphtalène comme cela est montré dans le tableau 3.

Dans le tableau 3, nous avons reporté les résultats obtenus avec et sans recyclage de tétraline, le procédé se déroulant selon l'exemple 1. La fraction résidu correspond à la fraction qui n'est pas le naphtalène et qui est récupéré lors de l'étape de cristallisation.

**Tableau 3**

| | | Sans recyclage | Avec recyclage |
|---|---|---|---|
| Entrée réacteur : | charge A | 100 | 97 |
| | tétraline | 0 | 3 |
| Sortie cristallisation : | Rendement naphtalène | 97 | 96.5 |
| | Pureté naphtalène | 99.96 | 99.96 |
| | Rendement tétraline | 3.0 | 0.5 |
| Rendement naphtalène | (sortie/entrée) | 97% | 99.5% |

Lorsqu'il n'y a pas de recyclage, le rendement en naphtalène est de 97%. Si l'on utilise le recyclage, le rendement en naphtalène est alors de 99.5%. Le rendement en naphtalène est alors notablement amélioré. On récupère 3.5% de tétraline. On effectue une purge d'environ 0.5% et l'on ne recycle que 3.0%. Cette purge, qui n'est pas obligatoire, a pour effet de maintenir la pureté du naphtalène à un niveau très élevé.

## Revendications

1. Procédé de traitement de coupe naphtalène contenant des impuretés soufrées et/ou azotées et/ou oxygénées et/ou oléfiniques, dans lequel, dans une première étape, la coupe naphtalène est mise au contact, en présence d'hydrogène, d'un catalyseur contenant au moins une matrice, au moins un élément du groupe VIII et au moins un élément du groupe VI
procédé caractérisé en ce que
a) le catalyseur de première étape utilisé contient 5-40% poids d'oxydes de métaux des groupes VIII et VI, avec un rapport pondéral en oxyde de métal du groupe VI sur oxyde de métal du groupe VIII compris entre 1.25 et 20, ledit catalyseur présentant une surface spécifique BET d'au plus 220 m²/g, un volume poreux compris entre 0.35 et 0.7 ml/g et un diamètre moyen de pores d'au moins 10 nm, et que la coupe naphtalène est mise au contact dudit catalyseur à une température de 150-325°C, sous une pression totale de 0.1 MPa-0.9 MPa, avec une vitesse volumique horaire de 0.05-10 h⁻¹ et le rapport molaire hydrogène/naphtalène est compris entre 0.1 et 1.3, de façon à ce que le rendement en tétraline soit inférieur à 10% poids, et
b) dans une seconde étape, une partie au moins de l'effluent issu de la première étape sont séparés de H₂S, NH₃ et l'eau, et
c) dans une troisième étape, une partie au moins de l'effluent issu de la seconde étape est soumise à un traitement permettant de séparer dudit effluent le naphtalène et la tétraline

2. Procédé selon la revendication 1, caractérisé en ce que la troisième étape a lieu par cristallisation

3. Procédé selon la revendication 1 caractérisé en ce que la troisième étape a lieu par distillation suivie d'une purification finale par cristallisation en milieu fondu

4. Procédé selon l'une des revendications précédentes, caractérisé en ce que au moins une partie de la tétraline séparée de la troisième étape est recyclée vers la première étape dans un rapport pondéral tétraline/naphtalène de 0.005 à 0.08

5. Procédé selon l'une des revendications précédentes, caractérisé en ce que le catalyseur de la première étape comprend au moins une matrice choisie dans le groupe formé par l'alumine, la silice-alumine, l'oxyde de bore, la magnésie, la zircone, l'argile, et au moins un métal du groupe VIII choisi dans le groupe formé par le cobalt et le nickel, et au moins un métal du groupe VI choisi dans le groupe formé par le molybdène et le tungstène

6. Procédé selon l'une des revendications précédentes, caractérisé en ce que le catalyseur de la première étape contient également du phosphore

7. Procédé selon la revendication 6, caractérisé en ce que la teneur en phosphore est inférieure à 15% poids (exprimée en P₂O₅)

8. Procédé selon l'une des revendications précédentes, caractérisé en ce que la surface BET du catalyseur est d'au plus 180 m²/g

9. Procédé selon l'une des revendications précédentes, caractérisé en ce que le diamètre moyen des pores est de 10-20 nm

10. Procédé selon l'une des revendications précédentes, caractérisé en ce que la seconde étape a lieu par strippage

11. Procédé selon l'une des revendications précédentes, caractérisé en ce que l'étape de cristallisation en milieu fondu opère à des température comprises entre 60-90° C pour obtenir une pureté de naphtalène supérieure à 99,90 %.

12. Procédé selon l'une des revendications précédentes, caractérisé en ce que 0-15% de volume poreux du catalyseur correspond à des pores de diamètre inférieur à 9 nm.

13. Procédé selon l'une des revendications précédentes, caractérisé en ce que dans la première étape, la coupe de naphtène est mise au contact du catalyseur de façon à réaliser une désulfuration d'au moins 98 %.

## Claims

1. A process for treating a naphthalene cut containing sulphur-containing and/or nitrogen-containing and/or oxygen-containing and/or olefinic impurities, in which, in a first step, the naphthalene cut is brought into contact in the presence of hydrogen with a catalyst containing at least one matrix, at least one group VIII element and at least one group VI element, said process being characterized in that:
a) the catalyst used in the first step contains 5-40% by weight of oxides of metals from groups VIII and VI, with a ratio of the oxide of the group VI metal to the oxide of the group VIII metal which is in the range 1.25 to 20 by weight, said catalyst having a BET specific surface area of at most 220 m²/g, a pore volume which is in the range 0.35 to 0.7 ml/g and an average pore diameter of at least 10 nm, and in that the naphthalene cut is brought into contact with said catalyst at a temperature of 150-325°C, at a total pressure of 0.1 MPa - 0.9 MPa, at an hourly space velocity of 0.05-10 h⁻¹ and with a hydrogen/naphthalene molar ratio which is in the range 0.1 to 1.3, such that the yield of tetralin is less than 10% by weight, and
b) in a second step, at least a portion of the effluent from the first step is separated from H₂S, NH₃ and water, and
c) in a third step, at least a portion of the effluent from the second step undergoes treatment to separate naphthalene and tetralin from said effluent.

2. A process according to claim 1, characterized in that the third step is a crystallisation step

3. A process according to claim 1, characterized in that the third step is a distillation step followed by final purification by melt crystallisation.

4. A process according to any one of the preceding claims, characterized in that at least a portion of the tetralin separated from the third step is recycled to the first step in a tetralin/naphthalene weight ratio of 0.005 to 0.08.

5. A process according to any one of the preceding claims, characterized in that the catalyst in the first step comprises at least one matrix selected from the group formed by alumina, silica-alumina, boron oxide, magnesia, zirconia, clay, and at least one group VIII metal selected from the group formed by cobalt and nickel, and at least one group VI metal selected from the group formed by molybdenum and tungsten.

6. A process according to any one of the preceding claims, characterized in that the catalyst used in the first step also contains phosphorous.

7. A process according to claim 6, characterized in that the phosphorous content is below 15% by weight (expressed as P₂O₅).

8. A process according to any one of the preceding claims, characterized in that the BET specific surface area of the catalyst is at most 180 m²/g.

9. A process according to any one of the preceding claims, characterized in that the average pore diameter is 10-20 nm.

10. A process according to any one of the preceding claims, characterized in that the second step is a stripping step.

11. A process according to any one of the preceding claims, characterized in that the melt crystallisation step is carried out at temperatures which are in the range 60-90°C to obtain a naphthalene purity of over 99.90%.

12. A process according to any one of the preceding claims, characterized in that 0-15 % of the porous volume corresponds to pores of diameter less than 9 nm.

13. A process according to any one of the preceding claims, characterized in that in the first step, the naphthalene cut is put in contact with catalyst such as to achieve at least 98 % desulfurization.

## Patentansprüche

1. Verfahren zum Behandeln von Naphthalin-Schnitten, die Unreinheiten wie Schwefel und/oder Stickstoff und/oder Sauerstoff und/oder Olefine, in welchem in einer ersten Stufe der Naphthalin-Schnitt bei Anwesenheit von Wasserstoff einen Katalysator kontaktiert, der aus wenigstens einer Matrix besteht, wenigstens ein Element der Gruppe Vlll und wenigstens ein Element der Gruppe VI enthält, dadurch gekennzeichnet, dass
a) der Katalysator in der ersten Stufe 5-40 Gew.-% Metalloxide der Gruppe Vlll und VI enthält, mit einem Gewichtsverhältnis an Metalloxid der Gruppe VI zu Metalloxid der Gruppe Vlll zwischen 1,25 und 20, wobei dieser Katalysator auf einer spezifischen BET-Oberfläche von höchstens 220 m²/g präsent ist, ein Porenvolumen zwischen 0,35 und 0,7 ml/g aufweist und einen mittleren Porendurchmesser von wenigstens 10 nm, und wobei der Naphthalin-Schnitt diesen Katalysator bei einer Temperatur von 150-325°C kontaktiert, unter einem Absolutdruck von 0,1 MPa-0,9 MPa, mit einer Volumenstundengeschwindigkeit von 0,05-10 h⁻¹ und einem Molverhältnis Wasserstoff/Naphthalin zwischen 0,1 und 1,3, derart, dass die Ausbeute an Tetralin niedriger als 10 Gew.-% beträgt, und
b) in einer zweiten Stufe wenigstens einer der Bestandteile, der fließend aus der ersten Stufe stammt, getrennt ist in H₂S, NH₃ und Wasser, und
c) in einer dritten Stufe wenigstens einer der Bestandteile, der fließend aus der zweiten Stufe stammt, einer Behandlung ausgesetzt ist, die zulässt, diesen fließend in Naphthalin und Tetralin zu trennen.

2. Verfahren nach Anspruch 1, dadurch gekennzeichnet, dass in der dritten Stufe die Kristallisation stattfindet.

3. Verfahren nach Anspruch 1, dadurch gekennzeichnet, dass in der dritten Stufe durch Destillation eine abschließende Purifikation durch Kristallisation im schmelzenden Medium folgt.

4. Verfahren nach einem der vorhergehenden Ansprüche, dadurch gekennzeichnet, dass wenigstanes ein Bestandteil des Tetralins in der dritten Stufe abgetrennt wird und gegen die erste Stufe in einem Gewichtsverhältnis von Tetralin zu Naphthalin 0.005 zu 0,08 zurückgeführt wird.

5. Verfahren nach einem der vorhergehenden Ansprüche, dadurch gekennzeichnet, dass der Katalysator der ersten Stufe aus wenigstens einer Matrix gewählt in eine Gruppe, die durch Aluminium, Silizium-Aluminium, Boroxid, Magnesium, Zirkonoxid, Ton gebildet ist, und wenigstens durch ein Metall der Gruppe Vlll gebildet ist, gewählt aus der Gruppe, die durch Kobalt und Nickel gebildet ist, und mindestens ein Metall der Gruppe VI, gewählt aus der Gruppe, die durch Molybdän und Wolfram gebildet ist.

6. Verfahren nach einem der vorhergehenden Ansprüche, dadurch gekennzeichnet, dass der Katalysator in der ersten Stufe aus Phosphor besteht.

7. Verfahren nach Anspruch 6, dadurch gekennzeichnet, dass der Gehalt an Phosphor weniger als 15 Gew.-% beträgt (ausgedrückt in P₂O₅).

8. Verfahren nach einem der vorhergehenden Ansprüche, dadurch gekennzeichnet, dass die BET-Oberfläche des Katalysators höchstens 180 m²/g ist.

9. Verfahren nach einem der vorhergehenden Ansprüche, dadurch gekennzeichnet, dass der mittlere Porendurchmesser 10-20 nm beträgt.

10. Verfahren nach einem der vorhergehenden Ansprüche, dadurch gekennzeichnet, dass in der zweiten Stufe das Strippen stattfindet.

11. Verfahren nach einem der vorhergehenden Ansprüche, dadurch gekennzeichnet, dass die Stufe der Kristallisation im schmelzenden Medium bei Temperaturen von 60-90°C stattfindet, um eine Reinheit des Naphthalins von mehr als 99,90% zu erreichen.

12. Verfahren nach einem der vorhergehenden Ansprüche, dadurch gekennzeichnet, dass 0-15% des Porenvolumens des Katalysators einem Porendurchmesser von weniger als 9 nm entsprechen.

13. Verfahren nach einem der vorhergehenden Ansprüche, dadurch gekennzeichnet, dass in der ersten Stufe der Naphthin-Schnitt den Katalysator kontaktiert, derart, dass eine Desulfurierung von mindestens 98% realisiert wird.
